(19) **Europäisches Patentamt / European Patent Office / Office européen des brevets**

(11) **EP 3 988 672 B1**

(12) **EUROPEAN PATENT SPECIFICATION**

(45) Date of publication and mention
of the grant of the patent:
**23.08.2023 Bulletin 2023/34**

(21) Application number: **21208315.8**

(22) Date of filing: **18.04.2018**

(51) International Patent Classification (IPC):
**C12Q 1/6869** (2018.01)     **C12Q 1/6883** (2018.01)

(52) Cooperative Patent Classification (CPC):
(C-Sets available)
**C12Q 1/6883; C12Q 1/6869;** C12Q 2600/156

(Cont.)

(54) **USE OF OFF-TARGET SEQUENCES FOR DNA ANALYSIS**

VERWENDUNG VON OFF-TARGET-SEQUENZEN ZUR DNA-ANALYSE

UTILISATION DE SÉQUENCES HORS CIBLE POUR L'ANALYSE D'ADN

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.04.2017 EP 17166836**

(43) Date of publication of application:
**27.04.2022 Bulletin 2022/17**

(62) Document number(s) of the earlier application(s) in
accordance with Art. 76 EPC:
**18717082.4 / 3 612 644**

(73) Proprietor: **Agilent Technologies, Inc.
Santa Clara, CA 95051 (US)**

(72) Inventor: **DEVOGELAERE, Benoit
1800 Vilvoorde (BE)**

(74) Representative: **Brantsandpatents bv
Pauline Van Pottelsberghelaan 24
9051 Ghent (BE)**

(56) References cited:
KR-B1- 101 721 480     US-A1- 2015 051 087
US-A1- 2015 066 824     US-A1- 2016 201 134

- **THOMAS KUILMAN ET AL: "CopywriteR: DNA copy number detection from off-target sequence data", GENOME BIOLOGY, BIOMED CENTRAL LTD., LONDON, GB, vol. 16, no. 1, 27 February 2015 (2015-02-27), page 49, XP021215490, ISSN: 1465-6906, DOI: 10.1186/S13059-015-0617-1**
- **EVANGELOS BELLOS ET AL: "cnvOffSeq: detecting intergenic copy number variation using off-target exome sequencing data", BIOINFORMATICS., vol. 30, no. 17, 1 September 2014 (2014-09-01), pages i639-i645, XP055390112, GB ISSN: 1367-4803, DOI: 10.1093/bioinformatics/btu475**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C12Q 1/6869, C12Q 2535/122**

**Description**

**Technical field**

The invention pertains to the technical field of genome analysis of a subject.

**Background**

Fetal aneuploidy and other chromosomal aberrations affect approximately 9 out of 1000 live births. Historically, the gold standard for diagnosing chromosomal abnormalities was karyotyping of fetal cells obtained via invasive procedures such as chorionic villus sampling and amniocentesis.

The discovery that significant amounts of cell-free fetal nucleic acids exist in maternal circulation has led to the development of new non-invasive prenatal genetic tests which allow for the detection of chromosomal aberrations.

Although a tremendous progress has been made in the field of clinical genetics over the last couple of years, there still remains a need for rapid, cost-effective, and more accurate diagnostic methods. Most currently available methodologies are based on the generation of very large amounts of genetic sequence data, whereby the majority of the information is non-essential or filtered out prior to diagnosis. The fact that for certain applications only a limited amount of genetic material is available indicates a need for methodologies that provide more accurate and effective analyses compared to those known in the art.

US 2015/066824 A1 describes a methodology wherein non-essential information generated during genetic sequencing is combined with the essential genetic sequencing data to predict the presence of polymorphisms in a subject from which the sample was taken. This method is, however, not suited to predict or monitor the health condition of a fetus, based on the analysis of a sample generated from the pregnant mother.

US 2015/051087 A1 discloses methods for determining a ploidy status of a chromosome in a gestating fetus from genotypic data a measured from a mixed sample of DNA comprising DNA from both the mother of the fetus and from the fetus, and optionally from genotypic data from the mother and father. The ploidy state is determined by using a joint distribution model to create a plurality of expected allele distributions for different possible fetal ploidy states given the parental genotypic data, and comparing the expected allelic distributions to the pattern of measured allelic distributions measured in the mixed sample, and choosing the ploidy state whose expected allelic distribution pattern most closely matches the observed allelic distribution pattern. The method disclosed may be relevant for detecting LOH in tumor cells. The method however does not use off-target reads generated during genomic sequencing.

KR 101 721 480 B1 discloses a method and a system for detecting chromosomal abnormality. The method uses targeted sequencing and makes use of on-target and off-target data to detect the variation. However, the method does not disclose using only off-target data to detect the genomic variation.

In addition, loss of heterozygosity (LOH) is a chromosomal event that results in the loss of substantially an entire gene or allele and optionally also a portion of the surrounding chromosomal region, a chromosome arm or an entire chromosome. LOH can happen with reduction in copy number or without reduction in copy number and is an important feature of many human cancers which can indicate certain characteristics of a patient's particular cancer. Thus, there is a strong need for faster, more sensitive, and more accurate methods for genome wide screening for LOH for utilizing LOH information in treating cancer patients.

Kuilman et al. (2015) and Bellos et al. (2014) both describe methods wherein non-essential information generated during genetic sequencing is used for the detection of DNA copy number variations in a subject. Seeing that not all LOH events give rise to a copy number alteration, these methods are not suited for accurate genome wide screening of LOH events in a subject.

In various embodiments, the present teachings make use of what has conventionally been considered non-informative, extraneous, or discarded data for diagnostic purposes. The methods described herein are particularly suitable for performing cell-free nucleic acid analysis applicable to prenatal diagnoses and tumor analysis, but may also readily be employed in other fields where aneuploidies and genetic aberration play an important role in the development of diseases or syndromes.

**Summary of the invention**

The invention is defined by the appended claims.

A method according to claim 1 is disclosed. The teachings provide methodologies for genomic or nucleic acid sequence analysis of biological samples from one or more subjects making use of off-target reads that may reside outside of a targeted or selected region generated for example from targeted-capture methods that make use of massively parallel sequencing technologies. The methodology allows the usage of nucleic acid sequencing information that may in other contexts be regarded as non-informative or extraneous genetic information. According to these methods, such sequence information may instead be advantageously leveraged to derive significant and even crucial information on the status of the sample from which the sequence reads and data are obtained. This includes information for example relating to aneuploidies and loss of heterozygosity (LOH) events. In various embodiments, by combining such off-target sequence data with that obtained from on-target sequence data, the extracted nucleic acids from a sample may be more efficiently used, reducing overall amounts of sample and downstream handling requirements. Such enhancements to existing sample processing and sequence analysis workflows are especially important in the field of cell-free analysis (including applications such as fetal chromosomal assessments and circulating tumor analysis). In such applications typically small or only very limited amounts of genetic material may be available and it is therefore a desirable aspect of the present teachings to more fully utilize sample sequence data to derive additional analytical or diagnostic insights considering both off-target and on-target sequence information.

## Detailed description of the invention

Disclosed are methodologies for sequence analysis that may be used in applications including genome analysis of a subject by evaluating sequence data associated with off-target reads generated for example when performing sample analysis by targeted-capture massively parallel sequencing methods. Such off-target sequence reads are often considered non-informative and overlooked or discarded. The inventor of the current technology and applications demonstrates that by leveraging off-target reads in sequence data useful insights and improvements useful for the detection of chromosomal aberrances, e.g. for fetal aneuploidy. The off-target reads also provide a useful tool for other sequence analysis applications including the genome-wide detection of loss of heterozygosity (LOH) which may be very difficult if not impossible with the currently available techniques especially in the context of shallow sequencing protocols.

Unless otherwise defined, all terms used in disclosing the innovative aspects of the present teachings, including technical and scientific terms, have the meaning as commonly understood by one of ordinary skill in the art to which the invention pertains. By means of further guidance, term definitions are included to better appreciate the teaching of the present invention.

As used herein, the following terms have the following meanings:

"A", "an", and "the" as used herein refers to both singular and plural referents unless the context clearly dictates otherwise. By way of example, "a compartment" refers to one or more than one compartment.

"About" as used herein referring to a quantifiable or measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints.

The expression "% by weight", "weight percent", "%wt" or "wt%", here and throughout the description unless otherwise defined, refers to the relative weight of the respective component based on the overall weight of the formulation.

The term "biological sample" as used herein refers to any sample that is obtained from or related to a subject (e.g., a human, such as a pregnant woman or other biological organism) and contains one or more nucleic acid molecule(s) of interest.

The term "massively parallel sequencing" or "next-generation sequencing" refers to technologies used in high throughput approaches for sequencing nucleic acids, including DNA, on the basis of generated sequencing libraries.

The term "targeted-capture massively parallel sequencing" refers to those massively parallel sequencing technologies whereby the nucleic acid samples to be sequenced may be enriched by means of a targeted capture step, said targeted capture could be performed on the basis of any suitable means, such as RNA or DNA probes. Such enrichment methods may be used to reduce the overall amount, number, or complexity of targets or fragments to be sequenced, reducing the overall difficulty or cost of the analysis by examining selected or desired target genetic (e.g. chromosomal) regions.

The term "panel", "probe" or "bait" in relation to the technique of targeted capture may include a molecule, moiety, or region used for targeting or selecting desired nucleic acid fragments (e.g. fragments or regions having a particular sequence, homology, or affinity) or interrogating selected genetic regions according to a particular targeted capture protocol.

The term "off-target reads" is to be understood as those reads which are obtained by the process of massively parallel sequencing for which targeted-capture of selected sequences result in a portion of non-specific sequence fragments or aspecific pairing of an amount of probe or bait with the nucleic acid sample, hence outside the expected panel, probe or bait, for example due to imperfect hybridization of the probe with the DNA.

The term "on-target reads" is to be understood as those sequencing reads which are obtained by a targeted-capture massively parallel sequencing process and which are the result of expected or specific pairing of the used panel, probe, or bait with the sample nucleic acids, hence in correspondence with the capture panel probe or bait.

The term "maternal sample" herein refers to a biological sample obtained from at least one pregnant subject e.g. a woman.

The term "subject" herein refers to a human subject as well as a non-human subject or a biological organism such as a mammal, an invertebrate, a vertebrate, a fungus, a yeast, a bacteria, and a virus. Although the examples herein concern human genomes and the language is primarily directed to human concerns, it will be appreciated that the present teachings are applicable to genomes from any biological organism, plant or animal, and may be useful in a variety of fields including but not limited to veterinary medicine, animal sciences, and research laboratories.

The term "biological fluid" herein refers to a liquid taken from a biological source and includes, for example, blood, serum, plasma, sputum, lavage fluid, cerebrospinal fluid, urine, semen, sweat, tears, saliva, blastocoel fluid and the like. It also refers to the medium in which biological samples can be grown, like in vitro culture medium in which cells, tissue or embryo can be cultured. As used herein, the terms "blood," "plasma" and "serum" expressly encompass fractions or processed portions thereof. Similarly, where a sample is taken from a biopsy, swab, smear, etc., the "sample" expressly encompasses a processed fraction or portion derived from the biopsy, swab, smear, etc.

The terms "maternal nucleic acids" and "fetal nucleic acids" herein refer to the nucleic acids of a pregnant female subject and the nucleic acids of the fetus being carried by the pregnant female, respectively. As explained before, "fetal nucleic acids" and "placental nucleic acids" are often used to refer to the same type of nucleic acids, though biological differences may exist between the two types of nucleic acids.

The term "fetal fraction" as used herein refers to the fractional representation or concentration of fetal nucleic acids present in a sample comprising fetal and maternal nucleic acids.

The term "copy number variation" or "CNV" herein refers to variation in the number of copies of a nucleic acid sequence that is a few base pairs (bp) or larger present in a first or test sample in comparison with the copy number of the nucleic acid sequence present in a second or qualified sample. A "copy number variant" refers to the few bp or larger sequence of nucleic acid in which copy-number differences are found by comparison of a sequence of interest in test sample with that present in a qualified sample. Nonlimiting copy number variants/variations include deletions, including microdeletions, insertions, including microinsertions, duplications, and multiplications. CNVs may encompass chromosomal aneuploidies and partial aneuploidies.

The term "aneuploidy" herein refers to an imbalance of genetic material caused by a loss or gain of a whole chromosome, or portion of a chromosome. Aneuploidy refers to both chromosomal as well as subchromosomal imbalances, such as, but not limiting to deletions, microdeletions, insertions, microinsertions, copy number variations, duplications. Copy number variations may vary in size in the range of a few bp to multiple Mb, or in particular cases from 1 kb to multiple Mb. Large subchromosomal abnormalities that span a region of tens of MBs and/or correspond to a significant portion of a chromosome arm, can also be referred to as segmental aneuploidies.

The term "chromosomal aneuploidy" herein refers to an imbalance of genetic material caused by a loss or gain of a whole chromosome, and includes germline aneuploidy and mosaic aneuploidy.

The term "loss of heterozygosity or LOH" refers to a chromosomal event that results in the loss of substantially an entire gene or allele and optionally also a portion of the surrounding chromosomal region, a chromosome arm or an entire chromosome.

The term "read" refers to an experimentally obtained DNA sequence whose composition and length (e.g., from about 20 bp or more) can be used to identify a larger sequence or region, e.g. a sequence portion or fragment that can be aligned and specifically assigned to a chromosome location or genomic region or gene. The terms 'read', 'sequence read' and 'sequences' may be used interchangeably throughout the specification.

The term "read count" refers to the number of reads associated with a sample that may be mapped to a reference sequence such as a genomic reference or a portion of said reference genome (read counts may be binned or grouped together on the basis of the location they map to with respect to a reference).

The term "reference genome" or ""reference sequence" as used herein refers to predetermined or sequence information distinct from a sample such as that contained in a digital nucleic acid sequence database. A reference genome or sequence may be a collection or assembly of sequence information representative of at least a portion of the nucleic acid sequences associated with a selected biological organism or species nucleic acids. A reference genome or sequence may be assembled from sequencing of nucleic acids from multiple samples and therefore, a reference genome or sequence does not necessarily represent the exact composition of a singular biological organism. In various embodiments, such references may be used to enable mapping of sequencing reads from one or more samples to specific or target chromosomal or genetic sequence positions.

The term "test sample" herein refers to a sample comprising a plurality or mixture of nucleic acids comprising at least one nucleic acid sequence whose copy number is suspected of having undergone variation or at least one nucleic acid sequence for which it is desired to determine whether a copy number variation exists. Nucleic acids present in a test sample are referred to as test nucleic acids or target nucleic acids or target chromosomes or target chromosomal segments.

The term "reference sample" herein refers to a sample comprising a plurality or mixture of nucleic acids from which the sequencing data are used along with the test sample sequencing data to analyze or calculate scores and parameters as described herein below and within the claims. In various embodiments, though not necessary, a reference sample is preferably normal or wild type (e.g. non-aneuploid) for the sequence of interest. In aneuploidy analysis, a reference sample may be a qualified sample that does not include sequences indicative of an aneuploid state such as trisomy 21 and that can be used for identifying the presence of a aneuploidy such as trisomy 21 in a test sample.

The term "reference set" comprises a plurality of "reference samples".

The term "bin" of a genome is to be understood as a segment of the genome. A genome can be divided in several bins, either of a fixed or predetermined size or a variable size. A possible fixed bin size can be e.g. 10 kB, 20 kB, 30 kB, 40 kB, 50 kB, 60 kB, 70 kB, etc. in which kB stands for kilobasepairs, a unit that corresponds to 1000 basepairs.

The term "window" is to be understood as a plurality of bins.

The terms "aligned", "alignment", "mapped" or "aligning", "mapping" refer to one or more sequences that are identified as a match in terms of the order of their nucleic acid molecules to a known sequence from a reference genome. Such alignment can be done manually or by a computer algorithm, examples including the Efficient Local Alignment of Nucleotide Data (ELAND) computer program distributed as part of the Illumina Genomics Analysts pipeline. The matching of a sequence read in aligning can be a 100% sequence match or less than 100% (non-perfect match).

The term "parameter" herein refers to a numerical value that characterizes a quantitative data set and/or a numerical relationship between quantitative data sets.

The term "cutoff value" or "threshold" as used herein means a numerical value whose value is used to arbitrate between two or more states (e.g. diseased and non-diseased) of classification for a biological sample. For example, if a parameter is greater than the cutoff value, a first classification of the quantitative data is made (e.g. diseased state); or if the parameter is less than the cutoff value, a different classification of the quantitative data is made (e.g. non-diseased state).

The term "imbalance" as used herein means any significant deviation as defined by at least one cutoff value in a quantity of the clinically relevant nucleic acid sequence from a reference quantity. For example, the reference quantity could be a ratio of 3/5, and thus an imbalance would occur if the measured ratio is 1:1.

It is the object of the current invention to provide a genetic analysis methodology of a sample on the basis of off-target reads obtained during targeted-capture massively parallel sequencing. These off-target reads were found especially useful for performing comprehensive prenatal diagnosis, but are also useful for the detection of aberrations, in DNA such as aneuploidies, mutations or LOH, e.g. in cancer panels. By using the off-target reads - which are not taken into account in conventional methods- the limited amount of available DNA (especially when using cell-free DNA as starting point) and DNA-derived sequencing data is optimally used. Both off- and on-target reads can simultaneously be used for one or more analyses on one sample, thereby limiting the amount of required handling steps such as library preparation and next-generation sequencing (NGS) and/or the bio-informatic or computational processing steps which might otherwise focus on or only retain on-target reads. As such, the limited amount of material is used in a most optimal manner.

Disclosed is a method for determining the presence or absence of a fetal chromosomal aneuploidy or fetal loss of heterozygosity (LOH) in a biological sample obtained from a pregnant female. Said method comprises specifically the following steps:

- obtaining sequence information indicative of targeted-capture massively parallel sequencing of the biological sample comprising both maternal and fetal nucleic acids;

- determining the amount of off-target reads obtained during said targeted capture massively parallel sequencing; and

- deriving from said off-target read counts information for determining the absence or presence of a fetal aneuploidy or fetal LOH.

In detail, the method requires the obtaining of maternal and fetal DNA from a biological sample taken from the pregnant mother. This biological sample may be blood, but could also be saliva or serum or any other sample derived from the mother and useful for obtaining genetic data from both mother and fetus. The cell-free DNA in the sample is subjected to a targeted enrichment in order to obtain a subset of the DNA, prior to sequencing.

Various methodologies for the targeted enrichment are known in the art and include both hybrid capture methods and PCR based amplicon capture technologies. Examples of such methodologies include for instance Sureselect® from Agilent Inc., Nimblegen® from Roche Inc. and TruSEq® from Illumina Inc.. The methodology of targeted enrichment is typically based on the use of labeled nucleic acid or other molecular probes able to hybridize to or associate with desired, or expected regions within a genome or isolated nucleic acid. In a subsequent step, the non-hybridized probes are washed away and the hybridized probes are captured and isolated from the sample. This capturing is performed by the presence of a label. Said label is able to bind, associate or connect to a second molecule which enables the capture of both label and hybridized region. Suitable labels known in the art are e.g. biotin, which may bind to streptavidin or avidin.

In a subsequent step, the captured regions are amplified and sequenced. As such, DNA regions are isolated and enriched. Enrichment of DNA by the method described above will inherently result in the generation of both off- and on-target reads as hybridization is a sensitive yet imperfect process that captures large amounts of off-target fragments along with the intended fragments.

In one embodiment, the probes used in the methodology are specifically designed against pre-defined target regions. Suitable panels or baits for which probes may be developed include microdeletions, CNVs e.g. small recurrent CNVs or known repeated regions. In one embodiment, said probes are directed to one or more regions known to contain recurrent CNVs or regions flanking said recurrent CNVs.

In another embodiment, said probes are randomly designed and not targeted to a specific panel or bait.

The size of the bait or panel is preferably between 0.1 kB to 100 Mb, more preferably between 1 kb and 50 Mb, between 1kb and 10 Mb, between 10 kB and 1 Mb, even more preferably between 20 kB and 0.5 Mb.

Although off-target reads are technically due to an aspecific binding of probes, the inventors of the current invention observed a trend in the aspecific binding of the probes. In other words, the off-target reads are not completely random but influenced by the sequence of the probe used. As a consequence, a reference set from one or more reference samples may be built. Said set of reference samples (or also termed reference set) can be predefined or chosen by a user (e.g. selected from his/her own reference samples). By allowing the user the use of an own reference set, a user will be enabled to better capture the recurrent technical variation of his/her environment and its variables (e.g. different wet lab reagents or protocol, different NGS instrument or platform, etc.). Moreover, by use of a high level of automation, technical variation, e.g. linked to human handling, is reduced. In a preferred embodiment, said reference set comprises genomic information of 'healthy' samples that are expected or known to not contain (relevant) aneuploidies, LOH or other genomic aberrations.

For the purpose of the current invention, the amount of the off-target read counts should be at least $1 \times 10^6$, more preferably at least $2 \times 10^6$, $3 \times 10^6$, $4 \times 10^6$, $5 \times 10^6$, $6 \times 10^6$, $7 \times 10^6$, $8 \times 10^6$, $9 \times 10^6$, $10 \times 10^6$ read counts.

Said sequences are obtained by next generation sequencing. By preference, a sequencing method with high coverage is used, also called deep sequencing. In a further preferred embodiment, a total of between $1\times10^6$ and $100 \times 10^6$ reads are generated, more preferably between $10 \times 10^6$ and $50 \times 10^6$ reads, even more preferably between $15 \times 10^6$ and $30 \times 10^6$ reads such as $20 \times 10^6$ reads.

Both paired-end read and single-reads may be used in the current technology

By preference, single-read NGS is used as single-read sequencing enables a lower sequencing cost.

After obtaining the NGS reads from said targeted-capture massively parallel sequencing, the reads are mapped to a reference genome or a portion of a reference genome (bin). Said mapping occurs by aligning the reads to said reference genome.

Subsequently, off-target and on-target reads are separated, thereby isolating the off-target reads. By preference, the identification or isolation of the off-target reads is done by an automated manner, e.g. by use of appropriate software known to the skilled in the art and that takes the targeted regions of the probes into account.

The read counts for the off-target reads are determined. In another or further embodiment, the read counts for both the on- and off-targets are determined. The total amount of reads for both the on- and/or off-target reads may be further subdivided based on their location within the reference genome, bin or window. By preference, the read counts are determined per bin.

In a further step, once obtained, the read counts may optionally be normalized. The reads could be normalized for the overall number of reads, whereby the samples are set to a predefined amount of reads (e.g. $1 \times 10^6$ reads or more). In another or further embodiment, normalization may occur on the basis of a set of reference samples, whereby said reference samples are preferably, though not necessary, euploid or essentially euploid. Such reference set may have various sample sizes. A possible sample size can be e.g. 100 samples, such as 50 male and 50 female samples. It will be understood by a skilled person that the reference set can be freely chosen by the user. By preference, such normalization occurs on bin or window level.

By preference, said number of reads is recalibrated to correct for GC content and/or total number of reads obtained from said sample. GC bias is known to aggravate genome assembly. Various GC corrections are known in the art. In a preferred embodiment, said GC correction will be a LOESS regression. In one embodiment, a user of the methodology according to the current invention can be provided with the choice of various possible GC corrections.

A detailed explanation on GC correction can be found in WO2017/009372.

The off-target read counts can subsequently be used to derive information regarding the presence or absence of a fetal aneuploidy or fetal LOH, or the general presence of an LOH or aneuploidy (e.g. in cancer panels, see further).

The determination whether or not a fetal aneuploidy is present on the basis of the off-target reads can be done by any algorithm known in the art which is capable of detecting fetal aneuploidies or LOH on the basis of cell-free DNA. Such systems include the OneSight® algorithm of Agilent, VeriSeq™ of Illumina or MaterniT21® Plus of Sequenom. In general, all known algorithms which are able to derive a parameter from the obtained reads, whereby the parameter is indicative for the presence or absence of an aneuploidy, can be used.

A particularly suitable methodology is described in application WO2017/009372. In short, from the alignments and the obtained off target read counts or a derivative thereof, optionally corrected for GC content and/or total number of reads obtained from said sample, scores are calculated which eventually lead to a parameter allowing the determination of the presence of an aneuploidy in a sample. Said scores are normalized values derived from the read counts or mathematically modified read counts, whereby normalization occurs in view of the reference set as defined by the user. As such, each score is obtained by means of a comparison with the reference set. It is important to note that the current methodology does not require training of the data or knowledge of the ground truth. The analysis according to the present teachings may use the nature of the reference set and does not require any personal choices or preferences set by the end user. Moreover, it can be readily implemented by a user without the need for access to proprietary databases.

The term first score is used to refer to score linked to the off target read count for a target chromosome or a chromosomal segment. A collection of scores is a set of scores derived from a set of normalized number of reads that may include the normalized number of reads of said target chromosomal segment or chromosome. Preferably, said first score represents a Z score or standard score for a target chromosome or chromosomal segment. Preferably, said collection is derived from a set of Z scores obtained from a corresponding set of chromosomes or chromosomal segments that include said target chromosomal segment or chromosome.

Preferably, said first score represents a Z score or standard score for a target chromosome or chromosomal segment. Preferably, said collection is derived from a set of Z scores obtained from a corresponding set of chromosomes or chromosomal segments that include said target chromosomal segment or chromosome.

The first score and the collection of scores are calculated on the basis of the genomic representation of either a target chromosome or chromosomal segment, or all autosomes or chromosomes (or regions thereof) thereby including the target chromosome or chromosome segment.

Such scores can be calculated as follows:

$$Zi = \frac{GRi - \mu\,ref,i}{\sigma\,ref,i}$$

With i a window or a chromosome or a chromosome segment and ref referring to the reference set.

A summary statistic of said collection of scores can e.g. be calculated as the mean or median value of the individual scores. Another summary statistic of said collection of scores can be calculated as the standard deviation or median absolute deviation or mean absolute deviation of the individual scores.

Said parameter p may be calculated as a function of the first score and a derivative (e.g. summary statistic) of the collection of scores. In a preferred embodiment, said parameter will be a ratio or correlation between the first score corrected by the collection of scores (or a derivative thereof) and a derivative of said collection of scores.

In another embodiment, said parameter will be a ratio or correlation between the first score corrected by a summary statistic of a first collection of scores and a summary statistic of a different, second collection of scores, in which both collections of scores include the first score.

Said parameter p is preferably a ratio or correlation between the first score, corrected by a summary statistic of said collection of scores, and a summary statistic of said collection of scores. Preferably, the summary statistic is selected from the mean, median, standard deviation, median absolute deviation or mean absolute deviation. In one embodiment, said both used summary statistics in the function are the same. In another, more preferred embodiment, said summary statistics of the collection of scores differ in the numerator and denominator.

Typically, a suitable embodiment involves the following steps (after having obtained off-target sequences from a sequencing process on a biological sample).

- aligning said obtained sequences to a reference genome;

- counting the number of off target reads on a set of chromosomal segments and/or chromosomes thereby obtaining read counts;

- normalizing said off target read counts or a derivative thereof into a normalized number of reads;

- obtaining a first score and a collection of scores of said normalized reads, whereby said first score is derived from the normalized reads for a target chromosome or chromosomal segment and said collection of scores is a set of scores derived from a corresponding set of chromosomes or chromosome segments that include said target chromosomal segment or chromosome;

- calculating a parameter p from said first score and said collection of scores, whereby said parameter represents a ratio or correlation between

  * said first score, corrected by a summary statistic of said collection of scores, and

  * a summary statistic of said collection of scores.

A possible parameter p can be calculated as follows:

$$Z \text{ of } Z_i = \frac{Z_i - \underset{j=i,a,b,\dots}{\text{median}}(Z_j)}{\underset{j=i,a,b,\dots}{\text{sd}}(Z_j)}$$

Whereby Zi represents the first score and Z j the collection of scores and whereby i represents the target chromosome or chromosomal section, and whereby j represents a collection chromosomes or chromosomal segments i, a, b, .. that include said target chromosomal segment or chromosome i.

In another embodiment, said parameter p is calculated as

$$Z \text{ of } Z_i = \frac{Z_i - \underset{j=i,a,b,\dots}{\text{mean}}(Z_j)}{\underset{j=i,a,b,\dots}{\text{mad}}(Z_j)}$$

Whereby Zi represents the first score and Z j the collection of scores and whereby i represents the target chromosome or chromosomal section, and whereby j represents a collection of chromosomes or chromosomal segments i, a, b, .. that includes said target chromosomal segment or chromosome i.

In yet another, most preferred embodiment, said parameter p is calculated as

$$Z \text{ of } Z_i = \frac{Z_i - \underset{j=i,a,b,\dots}{\text{median}}(Z_j)}{\underset{j=i,a,b,\dots}{\text{mad}}(Z_j)}$$

Whereby Zi represents the first score and Z j the collection of second scores and whereby i represents the target chromosome or chromosomal section, and whereby j represents a collection of chromosomes or chromosomal segments i, a, b, .. that includes said target chromosomal segment or chromosome i.

Said MAD for a data set $x\_1, x\_2, \dots, x\_n$ may be computed as

"MAD"=1.4826 × "median" (|x_i-"median" (x)|)

An alternative MAD that does not use the factor 1.4826 can also be used.

The factor 1.4826 is used to ensure that in case the variable x is normally distributed with a mean $\mu$ and a standard deviation $\sigma$ that the MAD score converges to $\sigma$ for large n. To ensure this, one can derive that the constant factor should equal $1/((\Phi^{-1}(3/4)))$, with $\Phi^{-1}$ is the inverse of the cumulative distribution function for the standard normal distribution.

The calculated parameter p, based on data obtained form off-target reads may subsequently be compared with a cutoff value for determining whether a change compared to a reference quantity exists (i.e. an imbalance), for example, with regards to the ratio of amounts of two chromosomal regions (or sets of regions). The cutoff value may be determined from any number of suitable ways. Such ways include Bayesian-type likelihood method, sequential probability ratio testing (SPRT), false discovery, confidence interval, receiver operating characteristic (ROC). Preferably, said cutoff value is based on statistical considerations or is empirically determined by testing biological samples. The cutoff value can be validated by means of test data or a validation set and can, if necessary, be amended whenever more data is available. In one embodiment, the user will be able to define its own cutoff value, either empirically on the basis of experience or previous experiments, or for instance based on standard statistical considerations. If a user would want to increase the sensitivity of the test, the user can lower the thresholds (i.e. bring them closer to 0). If a user would want to increase the specificity of the test, the user can increase the thresholds (i.e. bring them further apart from 0). A user will often need to find a balance between sensitivity and specificity, and this balance is often lab- and application -specific, hence it is convenient if a user can change the threshold values him- or herself.

Based on the comparison of the obtained parameter with the cutoff value, an aneuploidy may be found present or absent.

By preference, the methodology is particularly suitable for analyzing aneuploidies linked to segments or deletions given in Table 1, which contains a not-limiting list of chromosome abnormalities that can be potentially identified by methods and kits described herein.

In a further or other embodiment, the target chromosome is selected from chromosome X, Y, 6, 7, 8, 13, 14, 15, 16, 18, 21 and/or 22.

The methodology according to the current invention relates to evaluating the presence or absence of an LOH. The latter can be performed by using any algorithm known in the art capable of detecting changes in B-allele frequencies (BAF) across the set of positions that have sufficient coverage in the off-target reads. The method of the current invention is the first methodology which allows genome wide screening for LOH. This is specifically due to the nature of the off-target reads which are not completely random.

**Table 1**

| Chromosome | Abnormality | Disease Association |
|---|---|---|
| X | XO | Turner's Syndrome |
| Y | XXY | Klinefelter syndrome |
| | XYY | Double Y syndrome |
| | XXX | Trisomy X syndrome |
| | XXXX | Four X syndrome |
| | Xp21 deletion | Duchenne's/Becker syndrome, congenital adrenal hypoplasia, chronic granulomatous disease |
| | Xp22 deletion | Steroid sulfatase deficiency |
| | Xp26 deletion | X-linked lymph proliferative disease |
| 1 | 1p Monosomy, trisomy | |
| | 1p36 | 1p36 deletion syndrome |
| | 1q21.1 | 121.1 deletion syndrome; distal 1q21 deletion sydnrome |
| 2 | Monosomy, trisomy 2q | Growth retardation, developmental and mental delay, and minor physical abnormalities |
| | 2p15-16.1 | 2p15-16.1 deletion syndrome |
| | 2q23.1 | 2q23.1 deletion syndrome |
| | 2q37 | 2q37 deletion syndrome |
| 3 | Monosomy, trisomy | |
| | 3p | 3p deletion syndrome |
| | 3q29 | 3q29 deletion syndrome |
| 4 | Monosomy, trisomy | |
| | 4p- | Wolf-Hirschhorn syndrome |
| 5 | 5p | Cri du chat; Lejeune syndrome |
| | 5q Monosomy, trisomy | Myelodysplastic syndrome |
| | 5q35 | 5q35 deletion syndrome |

(continued)

| Chromosome | Abnormality | Disease Association |
|---|---|---|
| 6 | Monosomy, trisomy | |
| | 6p25 | 6p25 deletion syndrome |
| 7 | 7q11.23 deletion | William's syndrome |
| | Monosomy, trisomy | Monosomy 7 syndrome of childhood; myelodysplastic syndrome |
| 8 | 8q24.1 deletion | Langer-Giedion syndrome |
| | 8q22.1 | Nablus mask-like facial syndrome |
| | Monosomy, trisomy | Myelodysplastic syndrome; Warkany syndrome; |
| 9 | Monosomy 9p | Alfi's syndrome |
| | Monosomy 9p, partial trisomy 9p | Rethore syndrome |
| | trisomy | Complete trisomy 9 syndrome; mosaic trisomy 9 syndrome |
| | 9p22 | 9p22 deletion syndrome |
| | 9q34.3 | 9q34.3 deletion syndrome |
| 10 | Monosomy, trisomy | ALL or ANLL |
| | 10p14-p13 | DiGeorge's syndrome type II |
| 11 | 11p- | Aniridia; Wilms tumor |
| | 11p13 | Wagr syndrome |
| | 11p11.2 | Potocki Shaffer syndrome |
| | 11p15 | Beckwith-Wiedemann syndrome |
| | 11q- | Jacobsen syndrome |
| | Monosomy, trisomy | |
| 12 | Monosomy, trisomy | |
| 13 | 13q- | 13q-syndrome; Orbeli syndrome |
| | 13q14 deletion | |
| | Monosomy, trisomy | Patau's syndrome |
| 14 | Monosomy, trisomy | |
| 15 | 15q11-q13 deletion, monosomy | Prader-Willi, Angelman's syndrome |
| | Trisomy | |
| 16 | 16q13.3 deletion | Rubenstein-Taybi |
| | Monosomy, trisomy | |
| 17 | 17p- | 17p syndrome |
| | 17q11.2 deletion | Smith-Magenis |
| | 17q 13.3 | Miller-Dieker |
| | Monosomy, trisomy | |
| | 17p11.2-12 trisomy | Charcot-Marie Tooth Syndrome type 1; HNPP |
| 18 | 18p- | 18p partial monosomy syndrome or Grouchy Lamy Thieffry syndrome |
| | Monosomy, trisomy | Edwards Syndrome |

(continued)

| Chromosome | Abnormality | Disease Association |
|---|---|---|
| 19 | Monosomy, trisomy | |
| 20 | 20p- | Trisomy 20p syndrome |
| | 20p11.2-12 deletion | Alagille |
| | 20q- | |
| | Monosomy, trisomy | |
| 21 | Monosomy, trisomy | Down's syndrome |
| 22 | 22q11.2 deletion | DiGeorge's syndrome, velocardiofacial syndrome, conotruncal anomaly face syndrome, autosomal dominant Opitz G/BBB syndrome, Caylor cardiofacial syndrome |
| | Monosomy, trisomy | Complete trisomy 22 syndrome |

As the concentrations of cell-free DNA are typically low, and as a result, the amount of different genetic tests that can be performed on one sample is limited. The current invention allows the use of hitherto unemployed data for the generation of comprehensive genetic information.

Meanwhile, also the on target reads are available for further analysis of the sample, which enables maximal use of the sample. While the off-target reads may serve to analyze one or more clinical aspect of the sample, the on-target reads may be utilized to analyze one or more second clinical aspects of the same sample.

Hence, disclosed here is also a methodology for the detection of the presence or absence of a fetal aneuploidy and/or LOH as well as the determination of the fetal fraction and/or presence of microdeletions and/or aberrations on genetic information received from one sample, whereby the sample is subjected to targeted-capture massively parallel sequencing under the conditions described above, whereby the off-target (optionally combined with the on-target) read counts are used for the determination of the presence or absence of a fetal aneuploidy and/or LOH and whereby the on-target read counts are used for the determination of the fetal fraction and/or the presence of the microdeletions.

The determination of the fetal fraction on the basis of the on-target reads could be done by any algorithm known in the art which allows fetal fraction determination on the basis of single-end reads, in particular the methodology as described in PCT/EP2016/066621. In short, the determination of the fetal fraction relies on the determination of on-target read counts of sequences, preferably CNVs which are present in the fetus but not in the mother, or which are heterozygous in the mother. For the latter, probes are used during targeted-capture massively parallel sequencing which are preferably directed to a panel of known, recurrent CNVs having a relatively high frequency in the population. Whereas the on-target reads are used for the determination of the fetal fraction, the generated off-target reads are the basis for the determination of the presence of a fetal fraction and/or LOH.

Next to the determination of the fetal fraction, the detection of microdeletions and/or aberrations may also be based on the generation of on-target reads. By preference, the panel or bait may be chosen to cover a set of recurring microdeletions that are known to be clinically relevant. Optionally PCR duplicates could be eliminated during the library preparations step. Suitable tools for removal of duplicates include for instance the use of molecular barcodes and/or position-based de-duplication. The obtained on-target reads subsequently form the basis of the further detection of the presence or absence of microdeletions, based on algorithms known in the art.

Suitable microdeletions which may be analyzed via the current methodology are linked to syndromes including, but not limiting to DiGeorge syndrome, Prader-Willi syndrome, Angelman syndrome, Neurofibromatosis type 1, Neurofibromatosis type II, Williams syndrome, Miller-Dieker syndrome, Slith-Magenis syndrome, Rubinstein-Taybi syndrome, Wolf-Hirschhorn syndrome and Potocki-Lupski (1p36 deletion).

A suitable target panel may be directed to the regions which are known to be linked to the syndromes mentioned above.

To summarize, the methodology allows the user to generate information on the aneuploidy status and the presence of LOH in the DNA present in the cell-free fraction from a pregnant woman. Simultaneously, information on the fetal fraction and the presence of microdeletions may be obtained as well, all without the need to perform multiple library preparations from the limited amount of cell-free DNA. This has advantages a.o. because it does not require splitting up the sample to perform the library prep, which would further reduce the absolute amount of e.g. fetal DNA molecules that are present in the reaction mix.

The methodology is not limited to the detection of aneuploidies in the fetal field and on the basis of cell-free DNA. The current methodology can equally be used starting from genomic DNA, FFPE DNA or any other suitable type of DNA. As such, the current invention may be used for the general detection of aneuploidies and/or LOH events, for instance in the field of cancer detection, prevention and/or risk evaluation. The method of the current invention based on the generated off-target reads allows genome wide screening, which, especially for LOH, was hitherto not possible.

Disclosed, but not part of the claimed invention, is a method for detecting aneuploidies and/or loss-of-heterozygosity events (LOH) in a DNA sample obtained from a subject, said method includes

- targeted-capture massively parallel sequencing of said DNA;
- separating the off-target reads from the on-target reads;
- determining the amount of off-target reads obtained during said targeted capture massively parallel sequencing; and
- deriving from said off-target reads information for determining the absence or presence of said aneuploidy or LOH in said subject.

It will be obvious for a skilled person that the aspects as described above for the analysis of a maternal sample largely apply to this general methodology as well.

By preference, the methodologies as described above are all computer implemented. To that purpose, also disclosed here is a computer program product comprising a computer readable medium encoded with a plurality of instructions for controlling a computing system to perform an operation for performing a (prenatal) diagnosis of a (fetal) aneuploidy and/or screening for (fetal) aneuploidies, LOH, microdeletions and/or determination of the fetal fraction in a biological sample obtained from a subject, wherein the biological sample includes nucleic acid molecules.

Such operations comprise the steps of:

- receiving the sequences of at least a portion the nucleic acid molecules contained in a biological sample (either from a patient or a pregnant female)
- aligning said obtained sequences to a reference genome;
- separating the on-target reads from the off-target reads;
- counting the number of off-target reads and optionally the on-target reads;
- - normalizing said read counts or a derivative thereof into a normalized number of reads;
- calculating a parameter on the basis of the off-target reads, whereby said parameter is indicative for the presence of a (fetal) aneuploidy or LOH.

Said operations can be performed by a user or practitioner in an environment remote from the location of sample collection and/or the wet lab procedure, being the extraction of the nucleic acids from the biologic sample and the sequencing.

Said operations can be provided to the user by means of adapted software to be installed on a computer, or can be stored into the cloud.

After having performed the required or desired operation, the practitioner or user will be provided with a report or score, whereby said report or score provides information on the feature that has been analyzed. Preferably, report will comprise a link to a patient or sample ID that has been analyzed. Said report or score may provide information on the presence or absence of an aneuploidy or LOH in a sample, the presence or absence of microdeletions and when the sample is obtained from a pregnant female, the fetal fraction determination, whereby said information is obtained on the basis of a parameter which has been calculated by the above mentioned methodology. The report may equally provide information on the nature of the aneuploidy (if detected, e.g. large or small chromosomal aberrations) and/or on the quality of the sample that has been analyzed.

It shall be understood by a person skilled in the art that above-mentioned information may be presented to a practitioner in one report.

By preference, above mentioned operations are part of a digital platform which enables molecular analyzing of a sample by means of various computer implemented operations.

**Claims**

1. A method for genome-wide determination of the presence or absence of a loss of heterozygosity (LOH) in a biological sample, said sample comprising one or more DNA molecules, the method comprising:

    a) obtaining DNA from said biological sample;
    b) allowing hybridization of said DNA with one or more labeled nucleic acid probes;
    c) capturing said hybridized DNA:probes;
    d) performing sequencing of said captured DNA, thereby obtaining reads;

e) mapping said reads to a reference genome;
f) separating the on-and off-target reads;
g) obtaining off-target read counts;

and using said off-target read counts for determining the presence or absence of a LOH.

2. Method according to claim 1, further comprising normalizing said off-target reads on the basis of a reference set.

3. Method according to claim 1 or 2, whereby deep sequencing is performed.

4. Method according to any of the preceding claims, whereby the minimum amount of off-target read counts is between $1 \times 10^6$ and $100 \times 10^6$

5. Method according to any of the previous claims, wherein the biological sample comprises genomic DNA, FFPE DNA, or cell-free DNA.

6. Method according any of the previous claims, wherein the biological sample comprises a cancer panel sample.

7. Method according to any of the previous claims, **characterized in that** said probes are directed to a predefined target.

8. Method according to claim 7, **characterized in that** said probes are directed to repeated regions in said DNA or genomic regions.

9. Method according to any of the previous claims, **characterized in that** said probes are directed to random targets.

10. Method according to any of the previous claims, **characterized in that** said on-target reads are used for further analysis.


**Patentansprüche**

1. Verfahren zur genomweiten Bestimmung des Vorhandenseins oder Nichtvorhandenseins eines Verlusts der Heterozygotie (LOH) in einer biologischen Probe, wobei die Probe ein oder mehrere DNA-Moleküle umfasst, wobei das Verfahren Folgendes umfasst:

   a) Gewinnen von DNA aus der biologischen Probe,
   b) Ermöglichen der Hybridisierung der DNA mit einer oder mehreren markierten Nukleinsäuresonden,
   c) Erfassen der hybridisierten DNA-Sonden,
   d) Durchführen einer Sequenzierung der erfassten DNA, wodurch Reads gewonnen werden,
   e) Abbilden der Reads auf ein Referenzgenom,
   f) Separieren der On-Target- und Off-Target-Reads,
   g) Gewinnen von Zählwerten der Off-Target-Reads,

   und Verwenden der Zählwerte der Off-Target-Reads zum Bestimmen des Vorhandenseins oder Nichtvorhandenseins eines LOH.

2. Verfahren nach Anspruch 1, ferner das Normalisieren der Off-Target-Reads auf der Basis eines Referenzsatzes.

3. Verfahren nach Anspruch 1 oder 2, wobei eine Tiefensequenzierung durchgeführt wird.

4. Verfahren nach einem der vorhergehenden Ansprüche, wobei der minimale Betrag der Zählwerte der Off-Target-Reads zwischen $1 \times 10^6$ und $100 \times 10^6$ liegt.

5. Verfahren nach einem der vorhergehenden Ansprüche, wobei die biologische Probe Genom-DNA, FFPE-DNA oder zellfreie DNA umfasst.

6. Verfahren nach einem der vorhergehenden Ansprüche, wobei die biologische Probe eine Krebspanelprobe umfasst.

**7.** Verfahren nach einem der vorhergehenden Ansprüche, **dadurch gekennzeichnet, dass** die Sonden auf ein vordefiniertes Ziel gerichtet werden.

**8.** Verfahren nach Anspruch 7, **dadurch gekennzeichnet, dass** die Sonden auf sich wiederholende Regionen in der DNA oder den Genomregionen gerichtet werden.

**9.** Verfahren nach einem der vorhergehenden Ansprüche, **dadurch gekennzeichnet, dass** die Sonden auf zufällige Ziele gerichtet werden.

**10.** Verfahren nach einem der vorhergehenden Ansprüche, **dadurch gekennzeichnet, dass** On-Target-Reads für weitere Analysen verwendet werden.

**Revendications**

**1.** Procédé de détermination à l'échelle du génome de la présence ou de l'absence d'une perte d'hétérozygotie (LOH) dans un échantillon biologique, ledit échantillon comprenant une ou plusieurs molécules d'ADN, le procédé comprenant :

a) l'obtention d'ADN à partir dudit échantillon biologique ;
b) la réalisation de l'hybridation dudit ADN avec une ou plusieurs sondes d'acide nucléique marquées ;
c) la capture desdites sondes d'ADN hybridées ;
d) la réalisation du séquençage dudit ADN capturé, obtenant ainsi des lectures ;
e) le mappage desdites lectures sur un génome de référence ;
f) la séparation des lectures dans la cible et hors cible ;
g) l'obtention d'un nombre de lectures hors cible ;

et l'utilisation dudit nombre de lectures hors cible pour déterminer la présence ou l'absence d'une LOH.

**2.** Procédé selon la revendication 1, comprenant en outre la normalisation desdites lectures hors cible sur la base d'un ensemble de référence.

**3.** Procédé selon la revendication 1 ou 2, dans lequel un séquençage approfondi est effectué.

**4.** Procédé selon l'une quelconque des revendications précédentes, dans lequel la quantité minimale de lectures hors cible est comprise entre $1 \times 10^6$ et $100 \times 10^6$.

**5.** Procédé selon l'une quelconque des revendications précédentes, dans lequel l'échantillon biologique comprend de l'ADN génomique, de l'ADN FFPE, ou de l'ADN libre circulant.

**6.** Procédé selon l'une quelconque des revendications précédentes, dans lequel l'échantillon biologique comprend un échantillon de panel de cancer.

**7.** Procédé selon l'une quelconque des revendications précédentes, **caractérisé en ce que** lesdites sondes sont dirigées vers une cible prédéfinie.

**8.** Procédé selon la revendication 7, **caractérisé en ce que** lesdites sondes sont dirigées vers des régions répétées dans ledit ADN ou des régions génomiques.

**9.** Procédé selon l'une quelconque des revendications précédentes, **caractérisé en ce que** lesdites sondes sont dirigées vers des cibles aléatoires.

**10.** Procédé selon l'une quelconque des revendications précédentes, **caractérisé en ce que** lesdites lectures dans la cible sont utilisées pour une analyse ultérieure.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2015066824 A1
- US 2015051087 A1
- KR 101721480 B1
- WO 2017009372 A
- EP 2016066621 W